# EUROPÄISCHE PATENTSCHRIFT

(11) **EP 1 536 871 B1**
(45) Veröffentlichungstag und Bekanntmachung des Hinweises auf die Patenterteilung: **20.04.2016**
(21) Anmeldenummer: 03757844.0
(22) Anmeldetag: 15.09.2003
(51) Int. Cl.: B01D 9/00, C07C 51/43, B08B 3/04

(54) **WASCHVORRICHTUNG, EIN VERFAHREN ZUR AUFREINIGUNG EINES WASCHGUTS SOWIE DIE VERWENDUNG DER WASCHVORRICHTUNG**
WASHING DEVICE, METHOD FOR PURIFYING A WASHING PRODUCT, AND USE OF SAID WASHING DEVICE
DISPOSITIF DE LAVAGE, PROCEDE DE PURIFICATION D'UN PRODUIT LAVE, ET UTILISATION D'UN TEL DISPOSITIF DE LAVAGE

(30) Priorität: 13.09.2002 DE 10242746
(43) Veröffentlichungstag der Anmeldung: 08.06.2005
(73) Patentinhaber: Evonik Degussa GmbH, 45128 Essen (DE)
(72) Erfinder: NORDHOFF, Stefan, 45657 Recklinghausen (DE); BALDUF, Torsten, 45772 Marl (DE); DEINKEN, Joachim, 46286 Dorsten (DE); POHLISCH, Joachim, 63571 Gelnhausen (DE); HENGSTERMANN, Axel, 48308 Senden (DE)
(74) Vertreter: Lang, Arne
(86) Internationale Anmeldenummer: PCT/EP2003/010251
(87) Internationale Veröffentlichungsnummer: WO 2004/026429

(56) Entgegenhaltungen:
- EP-A- 0 175 401
- EP-A- 0 920 894
- WO-A-02/055469
- DE-A- 2 347 948
- US-A- 4 383 121
- US-A- 4 652 675
- US-A- 4 705 624
- US-A- 4 840 737

## Beschreibung

Die vorliegende Erfindung betrifft eine Waschvorrichtung, eine Reinigungsvorrichtung, eine Synthesevorrichtung, ein Verfahren zur Aufreinigung von einem Waschgut sowie die Verwendung einer Waschvorrichtung oder einer Reinigungsvorrichtung zur Aufreinigung und schließlich die Verwendung des durch die Aufreinigung erhaltenen Zielprodukts.

An die Reinheit von in der chemischen Industrie hergestellten Produkten werden immer höhere Anforderungen gestellt. Dieses gilt insbesondere für die sogenannten Feinchemikalien oder Pharmaerzeugnisse, die in vergleichsweise kleinen Mengen hergestellt werden. In jüngerer Zeit ist gleichfalls der Trend zu immer höheren Reinheitsanforderungen auch bei Chemikalien zu beobachten, die in sehr großen Mengen hergestellt werden. Bei diesen Substanzen handelt es sich meist um Ausgangsmaterialien, die in der weiteren Synthese von Massenpolymeren verwendet werden. In diesem Zusammenhang sind beispielsweise Acrylsäure, Methacrylsäure, Styrol, Acrylamid, Caprolactam, Naphthalin, oder Phenol zu nennen. Die hohen Reinheitsanforderungen gelten insbesondere dann, wenn die aus diesen Ausgangssubstanzen hergestellten Produkte im medizinischen, Lebensmittel- oder Hygienebereich eingesetzt werden. Beispielhaft für die Anwendungen im medizinischen und Hygienebereich seien im wesentlichen auf teilweise neutralisierten vernetzten Polyacrylsäuren basierenden absorbierenden Polymere genannt, die sowohl im medizinischen als auch im Hygienebereich in Form von Wundauflagen bzw. von Windeln zur Absorption von wässrigen Körperflüssigkeiten eingesetzt werden. Im Lebensmittelbereich werden beispielsweise bei der Trinkwasseraufarbeitung Flockungsmittel eingesetzt, die auf Acrylsäure oder Acrylamid oder beiden basieren.

Ein anderer Grund für die Reinheitsanforderungen an Ausgangsstoffen für Polymerisationen ist, dass diese Polymerisationen wesentlich kontrollierter ablaufen, wenn die eingesetzten Monomere in hoher Reinheit vorliegen. Auf diese Weise können die Molekulargewichte und Molekulargewichtsverteilungen, die maßgeblich sind für die Eigenschaften der aus diesen Monomeren synthetisierten Polymere, besser gesteuert werden.

Ein weiterer Aspekt in dem im industriellen Maßstab hohe Reinheiten gefordert werden, ist das Gebiet der Abwasseraufarbeitung. Bedingt durch die Tatsache, dass organische Lösemittel zunehmend mehr durch Wasser- oder wässrige Lösemittel bei der Technischen Synthese ersetzt werden, führt dazu, dass Abwasser von Syntheseprozessen in immer größeren Mengen anfallen. Das Gebot des Umweltschutzes fordert jedoch, dass diese Abwässer möglichst vollständig von den Produkten und Nebenprodukten der entsprechenden Synthese, in denen das Wasser als Lösemittel eingesetzt wurde, befreit werden.

Auch im Lebensmittelbereich steigen die Anforderungen an die Reinheit der für den Verzehr bestimmten Produkte immer mehr. Dieses gilt insbesondere bei der Konzentratherstellung. Zudem ist hierzu der Bedarf an preiswerten und das Lebensmittel schonenden Konzentrationsverfahren hoch.

Gleichfalls werden aufgrund der technischen Weiterentwicklung von Heizungen, Strahltriebwerken und Verbrennungsmotoren zur Leistungs- und Abgasoptimierung der Einsatz immer reinerer Kraftstoffe notwendig.

Bei der Synthese einer chemischen Verbindung oder bei der Gewinnung einer Substanz aus natürlichen Vorkommen fällt diese gewünschte Substanz jedoch üblicherweise nicht als Reinprodukt an. Vielmehr entsteht bei der Synthese oder bei der Gewinnung einer Substanz aus natürliche Vorkommen ein Verbindungsgemisch, dessen Teil die gewünschte Substanz neben Verunreinigungen wie Lösemittel, Ausgangsprodukte und Nebenprodukte oder unerwünschte Isomere enthält. Zur Abtrennung der gewünschten Substanz von den Verunreinigungen werden im industriellen Maßstab häufig destillative Trennverfahren eingesetzt, die jedoch mit einem hohen Energieaufwand verbunden sind und im Fall von thermisch sensiblen und meist reaktiven Endprodukten zu einer Ausbeuteverminderung führen, da die gewünschten Produkte auf Grund der vergleichsweise drastischen thermischen Bedingungen sich weiter umsetzen.

Handelt es sich bei der gewünschten Substanz um eine kristallisierbare Verbindung, die nach dem Syntheseprozess in einem flüssigen Verbindungsgemisch vorliegt, so bietet sich die Schmelzkristallisation als ein möglichst schonendes Verfahren zur Reinigung der gewünschten Substanzen, d. h. zum Abtrennen der Substanz aus dem flüssigen Verbindungsgemisch, oftmals als "Feed" bezeichnet, in dem weitere Nebenprodukte gelöst oder als Flüssigkeit vorliegen, an. Dabei wird die gewünschte Verbindung als Feststoff aus der Flüssigkeit auskristallisiert, anschließend der kristalline Feststoff von der restlichen Flüssigkeit, die allgemein als Mutterlauge bezeichnet wird, getrennt und wieder aufgeschmolzen. Die Schmelze wird dann als gereinigtes Produkt abgeführt.

Aus dem Stand der Technik sind als übliche Verfahren zur Kristallisation die statische und dynamische Schichtkristallisation, bei der die zu isolierende Verbindung an feststehenden, gekühlten Flächen abgeschieden wird, oder die Suspensionskristallisation, die auf dem Wachstum von Kristallen in einer Suspension beruht, bekannt. Die Suspensionskristallisation weist dabei gegenüber der Schichtkristallisation den Vorteil auf, dass diese in einem kontinuierlichen Prozess durchgeführt werden kann. Außerdem ist die Reinheit der Kristalle auf Grund ihrer vergleichsweise langsamen Wachstumsgeschwindigkeit in aller Regel sehr hoch. Gleichwohl kann trotz der langsamen Wachstumsgeschwindigkeit über die Suspensionskristallisation ein hoher Produktdurchsatz kontinuierlich erzielt werden, da der Kristallisation in Lösung eine vergleichsweise große Fläche -nämlich im die gesamte Kristalloberfläche aller Einzelpartikel - für das Kristallwachstum zur Verfügung steht. Folglich stellt die Suspensionskristallisation ein sehr wirksames und kostengünstiges Verfahren zur Erzielung hoher Reinheiten bei einem Zielprodukt da.

Auf Grund des gegenüber der Schichtkristallisation vergleichsweise langsamen Wachstums der Kristalle werden die in der Flüssigkeit befindlichen Verunreinigungen weitestgehend nicht in den Kristall eingebaut und in der Mutterlauge zurückbleiben. Bereits in einem einstufigen Kristallisationsprozess werden in der Regel hochreine Kristalle der gewünschten Verbindung erhalten.

Ein weiterer, für die Reinheit des Endproduktes wichtiger Schritt ist die Abtrennung der von der in der Suspension befindlichen Kristalle von den anderen flüssigen Bestandteilen der Suspension, die meist Verunreinigungen und die nicht kristallisierten Anteile des zu reinigenden Gemisches enthalten. Diese Abtrennung erfolg meist durch einen Fest/Flüssig-Trennprozess. Dieser Trennprozess kann ein oder mehrstufig ablaufen, wobei zumindest in der letzten Stufe üblicherweise eine sogenannte Waschkolonne als Waschvorrichtung verwendet wird. In einer derartigen Waschvorrichtung wird eine in einem Kristallisator erzeugte Kristallsuspension eingeleitet und die Kristallsuspension unter Ausbildung eines Kristallbetts verdichtet. Eine Waschflüssigkeit, vorzugsweise die Schmelze aus den aufgeschmolzenen Kristallen selbst, wird im Gegenstrom durch dieses Kristallbett geleitet.

Zur Ausbildung dieses kompakten Kristallbetts werden unterschiedliche Methoden eingesetzt. So beschreibt US Re. 24,038 eine gravimetrisch arbeitende Waschkolonne, der eine Kristallsuspension in einem oberen Bereich der Waschkolonne eingeführt wird und das Kristallbett sich auf Grund eines Sedimentationsprozesses ausbildet. Bei derartigen Kolonnen besteht jedoch die Gefahr, dass sich im Laufe des Sedimentationsprozesses vertikale Kanäle ausbilden, in denen eine Rückvermischung der Mutterlauge oder der Kristallsuspension mit der Waschflüssigkeit auftritt.

Wie DE OS 1947251 zu entnehmen ist, wurden die gravimetrisch arbeitenden Waschkolonnen zumindest auf einen Teil ihrer Höhe mit einem Rührwerk ausgestattet, dass die Ausbildung von vertikalen Flüssigkeitskanälen im Kristallbett verhindert. Derartige Rührwerke sind jedoch mit dem Nachteil verbunden, dass auf Grund der Rührbewegung eine Verwirbelung des Kristallbetts auftritt, die sich nachteilhaft auf die Trennleistung der Waschvorrichtung auswirkt.

Derartige Rühnverke sind bei hydraulischen oder mechanischen Waschkolonnen nicht erforderlich. So offenbart EP 0 920 894 A1 eine hydraulische Waschkolonne, bei der die Suspension unter Druck in ein druckdichtes Gehäuse befördert wird und sich durch den Förderdruck ein kompaktiertes Kristallbett ausbildet. Der Druck wird in EP 0 920 894 A1 durch einen semipermeablen Stempel erzeugt, der für die flüssige Phase der Kristallsuspension durchlässig ist. In DE OS 28 00 540 wird ein rotierendes Förderelement, das schneckenartig ausgebildet ist, als Maßnahme zur Kompaktierung der Kristalle zur Ausbildung des Kristallbetts eingesetzt.

Um die in dem Kristallbett verdichteten Kristalle wieder zu vereinzeln, um sie dem Bereich zuzuführen, in dem die Kristalle aufgeschmolzen werden, schlägt DE 100 39 025 A1 eine der Aufbaufront gegenüberliegend angeordnetes rotierendes Abtragwerkzeug vor, bei dem es sich in der Regel um ein Rotormesser oder einen Schaber handelt.

Bei diesen vorbeschriebenen beweglichen Maßnahmen zur Förderung bzw. Verdichtung der Kristalle zu einem Kristallbett und zur Abtrennung der Kristalle ist gemein, dass diese zu Inhomogenitäten in dem Kristallbett führen. Neben den signifikanten Kosten, die mit dem Einbau dieser beweglichen Teile in die Waschkolonnen anfallen, ist ein erheblicher Wartungsaufwand erforderlich. Die Wartung der beweglichen Teile in der Waschkolonne führt regelmäßig dazu, dass der Betrieb der Waschkolonne eingestellt, diese Teile ausgebaut, gereinigt, ausgebessert oder ersetzt werden müssen. Ein weiterer Nachteil stellen bewegliche Teile in der Waschkolonne insbesondere bei der Aufreinigung von reaktiven Substanzen dar. So tritt beispielsweise bei der Aufreinigung von Acrylsäure im Bereich der Dichtungen der Wellen der beweglichen Teile oftmals eine unerwünschte spontane Polymerisation auf, die dazu führt, dass der Betrieb der Waschkolonne eingestellt, die entstandenen Polymere entfernt und das bewegliche Teil ausgebaut, repariert oder ersetzt werden muss.

Zu dieser Fragestellung ist aus DE 37 86 471 T2 eine Waschkolonne bekannt, die im Bereich des Kristallbetts keine beweglichen Teile aufweist. Weiterhin offenbart DE 37 86 471 T2 verschiedene Lösungsansätze, das Verstopfen oder Blockieren der Einführungsöffnung zu verringern oder gar zu vermeiden. Diese Lösungsansätze berücksichtigen jedoch nicht die komplexen strömungsmechanischen Verhältnisse, die in dem Kristallbett und der Kristallsuspension sowie dem Übergangsbereich zwischen Kristallsuspension und Kristallbett herrschen. Diese Verhältnisse werden in Effect of Compressibility on Performance of Hydraulic Wash Columns, L. van Oord-Knol, O.S.L. Bruinsma, P.J. Jansens, AIChE Journal, July 2002, Col. 48, No. 7, Seiten 1478ff *und* A general Control Strategy For Hydraulic Packed Bed Wash Columns, P.J. Jansens. O.S.L. Bruinsma, G.M. van Rosmalen, R. de Goede, Trans IchemE, Vol. 72, Part A, September 1994, Seiten 695ff als bedeutend für den zuverlässigen Betrieb von Waschkolonne beschrieben.

Die US 4 652 675 A offenbart eine Waschvorrichtung gemäß dem Oberbegriff des Anspruchs 1.

Allgemein liegt dieser Erfindung somit die Aufgabe zu Grunde, die sich aus dem Stand der Technik ergebenen Nachteile abzumildern oder zu überwinden.

Insbesondere liegt eine erfindungsgemäße Aufgabe darin, eine Waschvorrichtung zur Verfügung zu stellen, die bei geeigneter Trennleistung möglichst kostengünstig und einfach herstellbar ist.

Zudem liegt eine erfindungsgemäße Aufgabe darin, eine Waschkolonne zur Verfügung zu stellen, die ohne weiteres für den großtechnischen Einsatz einem Upscaling unterzogen werden kann.

Ferner liegt eine erfindungsgemäße Aufgabe darin, eine Waschkolonne zu schaffen, die vergleichsweise lange Standzeiten hat, die insbesondere nicht durch zu häufige Reparaturarbeiten bedingte Ausfälle unterbrochen werden.

Zudem besteht eine erfindungsgemäße Aufgabe darin, ein Verfahren zur Aufreinigung eines Waschguts zur Verfügung zu stellen, das neben einer angemessenen Trennleistung einen möglichst kontinuierlichen und unterbrechungsfreien Betrieb gestattet und damit für den großtechnischen Einsatz besonders gut geeignet ist.

Die vorstehenden Aufgaben werden zum einen durch den Gegenstand des nachfolgenden Hauptanspruchs und sich aus den Unteransprüchen ergebenen Unterkombinationen gelöst.

Zudem betrifft die Erfindung eine Waschvorrichtung gemäß Anspruch 1, aufweisend
- einen Ersten Bereich, für die Zuführung eines Waschguts,
- einen Zweiten Bereich, für die Waschung des Waschguts, und
- einen Dritten Bereich, für die Aufschmelzung des Waschguts , sowie
- einen Strömungswiderstand, der zwischen dem Zweiten Bereich und dem Dritten Bereich vorgesehen ist,
wobei der Zweite Bereich mindestens teilweise aus einer Säule gebildet wird und wobei diese Säule einen Durchmesser von mindestens 300 mm aufweist und zwischen dem Ersten Bereich und dem Zweiten Bereich eine Fest/Flüssig-Trennvorrichtung vorgesehen ist. In der Regel ist der Durchmesser mit 8000 mm begrenzt.

Durch diese Dimensionierung des Zweiten Bereich wird der komplexen Strömungsmechanik des in der Waschvorrichtung bewegten Waschguts in vorteilhafter Weise entsprochen.

In einer Ausführungsform der erfindungsgemäßen Waschvorrichtung ist es bevorzugt, dass der Strömungswiderstand um eine zentrale Längsachse des Zweiten Bereichs drehfest vorgesehen ist. Es ist darüber hinaus bevorzugt, dass der Strömungswiederstand ortsfest zwischen dem Zweiten Bereich und dem Dritten Bereich vorgesehen ist. Besonders bevorzugt ist es, wenn der Strömungswiederstand ortsfest zwischen dem Zweiten Bereich und dem Dritten Bereich fest mit diesen Bereichen verbunden ist.

Als Waschgut ist eine Kristallsuspension bevorzugt. Eine derartige Kristallsuspension besteht ztun einen aus Kristallen und zum anderen aus einer flüssigen Phase. Es ist bevorzugt, dass die Kristalle zumindest 50 Gew.-%, vorzugsweise mindestens 75 Gew.-%, weiterhin bevorzugt mindestens 95 Gew.-% und besonders bevorzugt mindestens 99 Gew.-% sowie darüber hinaus bevorzugt mindestens 99,9 Gew.-% aus nur einem Zielprodukt bestehen. Grundsätzlich kann die erfindungsgemäße Waschvorrichtung zur Aufkonzentration und zur Fest/Flüssig-Trennung eingesetzt werden. Hierbei kann das Zielprodukt einerseits die bei der Fest/Flüssig-Trennung anfallende Flüssigkeit oder anderseits ein bei der Aufkonzentration anfallender kristallisierbare Feststoff sein. Allgemein können Zielprodukte Lebensmittel, Monomere, Treibstoffe, Lösemittel; oder zur Wasser der Abwasseraufarbeitung; oder Isomere, Diastereomere oder Enantiomere sein.

Als Lebensmittel sind Getränke bevorzugt. Als Monomere sind Acrylsäure, Methacrylsäure, Styrol, Methylmethacrylat, Butylacrylat, Benzoesäure, Bisphenol A, Caprolactam, Fettsäuren, Monochloressigsäure, Methyldiisocyanat, Toluylendisocyanat, Naphthalin, Paraffin, p-, o- oder m-Dichlorbenzol, p-Xylol, Phthalid oder jeweils deren Derivate oder jeweils deren Salze bevorzugt. Als Treibstoffe sind Hydrazin oder Diesel bevorzugt.

Monomere Zielprodukte sind vorzugsweise mindestens eine Doppelbindung aufweisende organische Verbindungen mit zwei bis 20 Kohlenstoffen oder Wasser. Als mindestens eine Doppelbindung aufweisende organische Verbindungen mit zwei bis 20 Kohlenstoffe sind Styrol, α-Methylstyrol, Acrylsäure, Methacrylsäure, Methylmethacrylat und Butylacrylat zu nennen, wobei Acrylsäure, Methacrylsäure, Methylmethacrylat bevorzugt und Acrylsäure besonders bevorzugt sind. Für den Fall, dass es sich bei der die Kristalle bildenden Zielprodukt um Wasser handelt, wird besonders bevorzugt Wasser eingesetzt, dass bei organischen Synthesen als Lösemittel anfällt und von den Haupt- und Nebenprodukten dieser organischen Synthesen abzutrennen ist. In diesem Zusammenhang kommen insbesondere Synthesen von mindestens eine Doppelbindung aufweisende organische Verbindungen, vorzugsweise Acrylsäure, Methacrylsäure, Acrolein oder Methacrylat, zu nennen, wobei die Synthesen von Acrolein oder Acrylsäure besonders bevorzugt sind.

Wenn das Waschgut als Gemisch einer festen und einer flüssigen Phase wie im Beispiel der Kristallsuspension vorliegt, ist es bevorzugt, dass die durch die Kristalle gebildete feste Phase einen Anteil im Bereich von 10 bis 80 Gew.-%, bevorzugt 15 bis 60 Gew.-% und darüber hinaus bevorzugt 20 bis 40 Gew.%, jeweils bezogen auf das gesamte Waschgut bzw. auf die gesamte Kristallsuspension, besitzt. Weiterhin weist das Waschgut eine flüssige Phase auf. Im oberen Teil des Zweiten Bereichs weist diese flüssige Phase vornehmlich eine aus den aufgeschmolzenen Kristallen stammende Waschflüssigkeit auf Im untern Teil des Zweiten Bereichs, vorzugsweise im Bereich der Fest/Flüssig-Trennvorrichtung, überwiegt in der flüssigen Phase die aus der Kristallbildung in einem Kristallerzeuger stammende Mutterlauge, die über die Fest/Flüssig-Trennvorrichtung abgeleitet werden kann.

Weiterhin ist es bevorzugt, dass das Waschgut, vorzugsweise die Kristallsuspension eine Viskosität von < 250 mPas, vorzugsweise < 100 mPas und besonders bevorzugt, < 50 mPas, bestimmt nach DIN 53019 ISO/ISO 3219, aufweist.

Wenn das Waschgut als Kristallsuspension vorliegt, ist es bevorzugt, dass die Kristalle einen mittleren Durchmesser von höchstens 1500 µm, vorzugsweise im Bereich von 50 bis 1000 µm, weiterhin bevorzugt im Bereich von 75 bis 500 µm und darüber hinaus bevorzugt im Bereich von 100 bis 250 µm aufweisen. Der mittlere Durchmesser wird anhand von 500 zufällig ausgewählten Kristallen der Kristallsuspension bestimmt. Die Größe eines Kristalls ist der Durchschnitt der größten Länge eines Kristalls und der zu der größten Länge rechwinkelig in deren Mitte gemessene Durchmesser des einzelnen Kristalls. Zur Bestimmung von Länge und Durchmesser wird Bildanalysesystem, bestehend aus einem Lichtmikroskop mit einer angeschlossenen CCD-Kamera und einer PC-Auswerteeinheit eingesetzt, wobei ein PC-Programm der Firma Soft Imaging Systems (SIS, V3.1) eingesetzt wird.

Weiterhin ist es bevorzugt, dass in der erfindungsgemäßen Waschvorrichtung mindestens im Zweiten Bereich ein Druck im Bereich von 0,1 bis 30 bar, vorzugsweise 0,5 bis 10 bar und besonders bevorzugt 1,5 bis 7 bar erreicht werden kann. Weiterhin ist es bevorzugt, dass der vorstehend genannte Druck höher ist, als in der Druck auf der von dem Waschgut abgewandten Seite des Filters.

So liegt beispielsweise bei einer Acrylsäure beinhaltenden Kristallsuspension die Konzentration der Acrylsäure in den Kristallen bei mindestens 90 Gew.-%, vorzugsweise mindestens 95 Gew.-% und besonders bevorzug mindestens 98 Gew.-% sowie weiterhin bevorzugt bei mindestens 99 Gew.-%, jeweils bezogen auf die Kristalle.

Besonders bevorzugt ist die Konzentration des aufgeschmolzenen Zielprodukts in der flüssigen Phase in dem Bereich des Strömungswiderstands im Vergleich zu von dem Strömungswiderstand entfernteren Bereichen des Zweiten Bereichs höher.

Bei der Aufarbeitung von Abwässern aus der Acrylsäure- oder Acrolein-Synthese, vorzugsweise aus der Acrolein-Synthese, bestehen die die Kristallsuspension bildenden Wasserkristalle zumindest 90 Gew.-%, vorzugsweise mindestens 99 Gew.-% und besonders bevorzugt mindestens 99,9 Gew.-%, jeweils bezogen auf die Kristalle, aus Wasser.

In der erfindungsgemäßen Waschvorrichtung ist es bevorzugt, dass der erste Bereich, dem ein Waschgut zugeführt wird, als Öffnung ausgebildet ist, an der sich vorzugsweise eine röhrenartige Leitung anschließt mit der mindestens ein Waschgutfördermittel verbunden ist, dass vorzugsweise außerhalb der Waschvorrichtung liegt. Vorteilhafterweise werden mehrerer Fördervorrichtungen für das Waschgut redundant eingesetzt. Dieses empfiehlt sich insbesondere dann, wenn das Waschgut reaktive Verbindungen beinhaltet. Auf diese Weise kann der Betrieb der erfindungsgemäßen Waschvorrichtung fortgesetzt werden, wenn eine der mehreren Fördervorrichtungen beispielsweise durch eine ungewollte spontane Polymerisation dieser reaktiven Verbindungen, ausfällt. Die Reparatur der ausgefallenen Fördervorrichtung kann einfach und bequem erfolgen, da diese Fördervorrichtung sich außerhalb der Waschvorrichtung befindet.

Als Fördervorrichtungen für die erfindungsgemäße Waschvorrichtung kommen alle dem Fachmann geeigneten Fördervorrichtungen in Betracht. Insbesondere sind Fördervorrichtungen bevorzugt, die das Waschgut möglichst gering mechanisch belasten, hierbei ist insbesondere eine geringe Scherung des Waschguts vorteilhaft. Zudem sollten die Fördereinrichtungen durch einen möglichst pulsationsarmen Betrieb zu möglichst wenig Störungen des Kristallbetts führen. Unter "pulsationsarm" wird verstanden, dass der Druck des Förderguts am Ausgang des Fördermittels um weniger als 0,5 bar schwankt. Vor diesem Hintergrund sind die kontinuierlich arbeitenden Schneckenförderpumpen und Drehkolbenpumpen, Kreiselpumpen, vorzugsweise mit möglichst großem Spaltabstand, vorzugsweise das mindestens 2-fache des mittleren Kristalldurchmessers, oder Mehrkolbenmembranpumpen gegenüber diskontinuierlich arbeitenden Hubkolbenpumpen bevorzugt, wobei Schneckenförderpumpen besonders bevorzugt sind. Daher ist in der erfindungsgemäßen Waschvorrichtung vor dem Ersten Bereich oder mindestens teilweise in dem Ersten Bereich ein pulsationsarmes Fördermittel vorgesehen, wobei das pulsationsarme Fördermittel vorzugsweise eine Förderschnecke aufweist.

Der Zweite Bereich der erfindungsgemäßen Waschvorrichtung, in dem das Waschgut gewaschen wird ist allgemein so ausgebildet und ausgestaltet, dass das Waschgut sich in diesem Bereich möglichst frei von Inhomogenitäten mit möglichst laminarer Strömung bewegen kann.

Es ist bevorzugt, dass sich beim Betrieb der erfindungsgemäßen Waschvorrichtung keine Aufbaufront in dem gesamten Zweiten Bereich befindet. Die Aufbaufront bezeichnet somit den Übergang von der Suspension zum Kristallbett und ist durch einen relativ abrupten Anstieg des Kristallgehalts in der Suspension gekennzeichnet. Darüber hinaus ist es bevorzugt, dass bei dem Betrieb der erfindungsgemäßen Waschvorrichtung die Aufbaufront sich in dem Ersten Bereich, vorzugsweise oberhalb der fördernden Teile der Fördervorrichtung, befindet.

Weiterhin ist es bevorzugt, dass bei einem röhrenförmig ausgestalteten Zweiten Bereich die Länge der Röhre um höchstens das 10-fache, bevorzugt höchsten 5-fache und besonders bevorzugt höchsten 1-fache größer ist als der Querschnitt der in dem Zweiten Bereich eingesetzten Röhre.

Der dritte Bereich der erfindungsgemäßen Waschvorrichtung, in dem das Waschgut aufgeschmolzen wird, ist vorzugsweise so ausgestaltet, dass sich keine Bereiche deutlicher Überhitzung bilden, durch die ungewollte Reaktionen von im Waschgut enthaltenden reaktiven Verbindungen erfolgen können. Genauso wie der Zweite Bereich ist auch der Dritte Bereich der erfindungsgemäßen Vorrichtung vorzugsweise röhrenförmig ausgebildet. Bei einer Ausführungsform des Dritten Bereichs befinden sich Heizelemente an der Wand des Dritten Bereichs. Gemäß einer anderen Ausführungsform des Dritten Bereichs befinden sich Heizelemente innerhalb des Dritten Bereichs. Ferner sind Kombinationen dieser beiden Ausfuhrungsformen möglich. Gemäß einer anderen Ausführungsform der Waschkolonne befindet sich ein Aufschmelzewärmetauscher alleine oder zusätzlich außerhalb des Dritten Bereichs. In diesem Fall wird die Erwärmung des Dritten Bereichs durch die Rückführung aufgeschmolzenen Zielprodukts erreicht. Dieser aus dem Dritten Bereich ausgelagerte Aufschmelzewärmetauscher ist bei besonders empfindlichen Substanzen bevorzugt, wobei die Oberflächentemperatur der Wärmeübertragungsflächen des Aufschmelzwärmetauschers vorzugsweise maximal 5°C, vorzugsweise maximal 3°C und besonders bevorzugt maximal 1,5°C über dem Schmelzpunkt des Zielproduktes liegt.

Der Strömungswiderstand ist so ausgebildet und ausgestaltet, dass dieser zunächst die in dem Waschgut beinhalteten festen Bestandteile, vorzugsweise die Kristalle einer Suspension, verdichtet, ohne dass diese den Grund der Verdichtung im Strömungswiderstand verstopfen. Damit ist es nicht notwendig, einen beweglichen Schaber oder Kratzer einzusetzen, der die kompaktierten Feststoffe des Waschguts zum Eintritt in den Dritten Bereich vereinzeln muss. Diese Vereinzelung erfolgt erfindungsgemäß durch den Strömungswiderstand.

Gemäß einer Ausuhrungsform der erfindungsgemäßen Waschvorrichtung ist es bevorzugt, dass der Strömungswiderstand einen durch eine relative freie Querschnittsfläche im Bereich von >0 bis <100 %, vorzugsweise im Bereich von 20 bis 80 % und besonders bevorzugt im Bereich von 40 bis 60 % bezogen auf die Gesamtfläche des Strömungswiederstands, gekennzeichnet ist.

Weiterhin ist es bevorzugt, dass in dem Strömungswiderstand vorgesehene Öffnungen einen Querschnitt im Bereich von 0,001 bis 50 mm, vorzugsweise im Bereich von 5 bis 25 mm und besonders bevorzugt im Bereich von 10 bis 20 mm aufweisen.

Es ist bevorzugt, dass in der erfindungsgemäßen Waschvorrichtung der Querschnitt der mindestens einen Öffnung variierbar ist.

Weiterhin ist es bevorzugt, dass die Wandungen der Öffnungen mit einer Flüssigkeit zur Verbesserung der Durchleitbarkeit des Waschguts benetzbar sind. Der auf den Wandungen der Öffnung wird hierzu ein Film ausgebildet, um diese zu Benetzen und das Anhaften der Kristalle vermindern oder gar verhindert. Dieser besteht vorzugsweise vornehmlich aus aufgeschmolzenem Zielprodukt, das über eine in dem Strömungswiderstand vorgesehene Heizvorrichtung aufgeschmolzen wird.

Diese Maßnahmen erlauben beispielsweise Druckschwankungen in dem Waschgut, insbesondere in dem Kristallbett, auszugleichen und dem Entstehen von Verstopfungen vorzubeugen.

Gemäß einer Ausführungsform sind diese Öffnungen mit einer verstellbaren Blende ausgestattet. Gemäß einer anderen Ausführungsform wird die Variierbarkeit des Querschnitts der Öffnung dadurch bewerkstelligt, in dem der Strömungswiderstand aus einer Öffnungen aufweisenden ersten Platte und einer weiteren drehbar zu der ersten Platte angeordneten, ebenfalls Öffnungen aufweisenden zweiten Platte besteht.

Gemäß einer weiteren Ausführungsform kann die mindestens eine Öffnung des Strömungswiderstands durch Streben eines Gitters gebildet werden. Die Stärke des Strömungswiderstands kann dadurch variiert werden, in dem mindestens zwei dieser Gitter beweglich zueinander angeordnet werden und durch gegeneinander Verschieben die in den Gittern ausgebildeten Öffnungen mehr oder weniger stark durch Streben des jeweils anderen Gitters überlagert werden.

Weiterhin kann es in der erfindungsgemäßen Waschvorrichtung bevorzugt sein, dass die flüssige Phase des Waschguts, beispielsweise aufgeschmolzene Kristalle, in einer das Waschgut bildenden Kristallsuspension aus der mindestens einen Bohrung des Strömungswiderstands austreten kann. Mit dieser Maßnahme kann dem Verstopfen der in dem Strömungswiderstand vorgesehenen Öffnungen vorgebeugt werden und die Trennleistung der Waschkolonne erhöht werden. Diese Bohrungen sind vorzugsweise kleiner als der mittlere Kristalldurchmesser.

Ferner ist es in einer Ausführungsform der erfindungsgemäßen Waschvorrichtung bevorzugt, dass ein dem Zweiten Bereich zugewandter A-Querschnitt der Öffnung um 10 größer ist, als der dem Dritten Bereich zugewandte B-Querschnitt. Vorzugsweise sind die Öffnungen konisch ausgebildet. Auch diese Maßnahme beugt der Verstopfung der Öffnungen und damit des Strömungswiderstandes vor. Außerdem erleichtert diese Maßnahme den Aufbau eines möglichst gleichmäßigen Kristallbetts. Hierbei ist es besonders bevorzugt, dass die dem Zweiten Bereich zugewandte Fläche des Strömungswiderstands konvexe Vertiefungen aufweiset, von denen die Öffnungen ausgehen.

Die erfindungsgemäße Waschvorrichtung weist zwischen dem Ersten Bereich und dem Zweiten Bereich eine Fest/Flüssig-Trennvorrichtung, vorzugsweise ein Filter, mit einer Filtratableitung auf. Weiterhin ist es bevorzugt, dass die Konzentration des Zielprodukts in der Filtratableitung gleich, vorzugsweise geringer ist, als die Konzentration des Zielprodukts in der Flüssigen Phase im Ersten Bereich. Zudem ist es bevorzugt, dass die Konzentration des Zielprodukts im Dritten Bereich größer als im Zweiten Bereich ist.

Als geeignete Filter können diejenigen Filtermaterialien eingesetzt werden, die eine Porengröße aufweisen, die kleiner ist als die mittlere Teilchengröße der in dem Waschgut beinhalteten festen Bestandteile. Es ist bevorzugt, dass die mittlere Porengröße der Filtermaterialien im Bereich von 1 bis 1000 µm, vorzugsweise im Bereich von 50 bis 800 µm und besonders bevorzugt im Bereich von 100 bis 500 µm liegen. Geeignete Filtermaterialien sind unter anderem Drahtgitter, -gewebe, - gelege oder -gewirke.

Insofern ist es in der erfindungsgemäßen Waschvorrichtung bevorzugt, dass der Filter in einer an den Zweiten Bereich sich anschließenden Wandung ausgebildet ist. Zudem ist es bevorzugt, dass die Wandung, in der der Filter ausgebildet ist, mit einem Winkel α im Bereich von >0 und <90°, vorzugsweise von 1 bis 50° oder bevorzugt von 15 bis 60° und besonders bevorzugt von 10 bis 30° oder 5 bis 15°, bezogen auf die durch die Strömungsrichtung des die Waschvorrichtung durchströmenden Waschguts gebildeten Achse in der Waschvorrichtung angeordnet ist.

Zudem ist es bevorzugt, dass zumindest der Zweite Bereich der erfindungsgemäßen Waschvorrichtung , vorzugsweise der Filter, temperierbar ist.

Weiterhin betrifft die Erfindung eine Reinigungsvorrichtung, aufweisend einen mit dem Ersten Bereich der vorstehend definierten Waschvorrichtung kristallleitend verbundenen Kristallerzeuger. Als Kristallerzeuger kommen grundsätzlich alle dem Fachmann zur Erzeugung einer Kristallsuspension geeigneten Vorrichtungen in Betracht. In diesem Zusammenhang werden beispielhaft Kühlscheibenkristaller genannt.

Die Erfindung betrifft ferner eine Reinigungsvorrichtung, aufweisend einen mit
der erfindungsgemäßen Waschvorrichtung kristallleitend verbundenen Kristallerzeuger.

Gemäß einer bevorzugten Ausführungsform der erfindungsgemäßen Reinigungsvorrichtung kann zwischen dem Kristallerzeuger und der Waschvorrichtung ein Verweilzeitbehälter beispielsweise zur Reifung oder zur Homogenisierung der Kristallsuspension vorgesehen sein.

Gemäß einer anderen Ausführungsform der Reinigungsvorrichtung sieht eine zumindest teilweise Rückführung der über den Filter abgetrennten Mutterlauge in die Kristallsuspension vor. Dieses kann vor oder nach, vorzugsweise nach der Fördervorrichtung und besonders bevorzugt zwischen Fördervorrichtung und Fest/Flüssig-Trennung erfolgen.

Gemäß einer weiteren Ausführungsform der Reinigungsvorrichtung weist diese eine zumindest teilweise Rückführung des im Dritten Bereich aufgeschmolzenen Reinprodukts in den Dritten Bereich vor.

Zudem weist eine andere Ausführungsform der Reinigungsvorrichtung eine zumindest teilweise Rückführung des in dem Dritten Bereich aufgeschmolzenen Reinprodukts in den Strömungswiderstand vor.

Ferner weist eine weitere Ausführungsform der Reinigungsvorrichtung eine zumindest teilweise Rückführung des in dem Dritten Bereich aufgeschmolzenen Reinprodukts in den Dritten Bereich auf, wobei eine Verwirbelung der in dem Dritten Bereich vorhandenen Kristalle erzeugt wird.

Die in der Reinigungsvorrichtung vorgesehenen Hauptströme, Rückführungen sowie die Variierung der Öffnungen des Strömungswidersands lassen sich anhand mittels eines, vorzugsweise einstückigen, Multithermofühlers oder Multiwiderstandsthermometer an mindestens einer Stelle gewonnener Daten steuern. Vorzugsweise ist der Multithermofühler im zentralen Bereich des, vorzugsweise rotationssymetrischen, Kristallbetts angeordnet. Diese Steuerung dient dem Zweck einen möglichst gleichmäßigen Waschgutfluss und eine möglichst hohe Trennleistung zu erzielen. Dieses erfolgt insbesondere durch die über die Temperaturmessung gesteuerten Hauptströme und Rückführungen, über die die Position der Waschfront festgelegt werden kann. Außerdem lassen sich so die Druckverhältnisse in der Waschkolonne regeln. Einzelheiten hierzu ergeben sich aus DE 100 39 025 A1, DE 100 36 880 A1 und aus DE 100 36 881. Die durch mehrere Messsonden hervorgerufenen Inhomogenitäten des Kristallbetts werden hierdurch bei Gewährleistung der Steuerung der Position der Waschfront vermieden.

Ferner ist es bevorzugt, dass mindestens zwei erfindungsgemäße Reinigungsvorrichtungen in Serie oder parallel zu einander verschaltet sein können. Die Art der Verschaltung und die Zahl der in einer Verschaltung verwendeten Reinigungsvorrichtungen oder Waschvorrichtungen richtet sich nach den Reinheitsanforderungen und dem Aufreinigungspotential des Zielprodukts.

Darüber hinaus betrifft die vorliegende Erfindung eine Synthesevorrichtung, aufweisend einen Syntheseeinrichtung, vorzugsweise einen Reaktor, und Stromab eine zuvor definierte Reinigungsvorrichtung. Im Zusammenhang mit der Synthese von Acrylsäure und Methacrylsäure und den bevorzugten Kombinationen von Kristallerzeugern und Waschvorrichtungen wird auf die Ausführungen in WO 02/055469 verwiesen. Es ist somit in einer erfindungsgemäßen Ausführungsform bevorzugt, dass die Syntheseeinrichtung eine Gasphasenoxidationssyntheseeinheit ist. Im Zusammenhang mit der Herstellung von polymerisierbaren Stoffen, insbesondere die Monomere Acrolein, (Meth)Acrylsäure, insbesondere Acrylsäure, wird auf *"*Acrolein und Derivate" in "Stets geforscht", Band 1 und 2, Chemieforschung im Degussa-Forschungszentrum Wolfgang 1988, Seiten 108 bis 126 verwiesen.

Ferner betrifft die Erfindung ein Verfahren zur Aufreinigung von einem Waschgut, wobei das Waschgut über den Ersten Bereich einer zuvor definierten Waschvorrichtung zugeführt wird.

In einer bevorzugten Ausführungsform des erfindungsgemäßen Verfahrens beinhaltet das Waschgut mindestens 20 Gew.-%, bevorzugt mindestens 50 Gew.-% und besonders bevorzugt mindestens 85 Gew.-% des Zielproduktes, vorzugsweise Acrylsäure.

Zudem betrifft die Erfindung die Verwendung des nach einem erfindungsgemäßen Verfahrens erhältliche Zielprodukt bei der Herstellung von Lebensmitteln, Polymeren, Treibstoffen, Schmiermitteln, Reinigungsmitteln, Farbstoffen oder Arzneimitteln.

Schließlich betrifft die Erfindung die Verwendung einer erfindungsgemäßen Waschvorrichtung oder einer erfindungsgemäßen Reinigungsvorrichtung zur Aufreinigung von Zielprodukten wie Lebensmittel, Monomere, Treibstoffe, Lösemittel; oder zur Abwasseraufarbeitung; oder zur Isomerentrennung.

Als Lebensmittel sind Getränke bevorzugt. Als Monomere sind Acrylsäure, Methacrylsäure, Styrol, Methylmethacrylat, Butylacrylat, Benzoesäure, Bisphenol A, Caprolactam, Fettsäuren, Monochloressigsäure, Methyldiisocyanat, Toluylendisocyanat, Naphthalin, Paraffin, p-, o- oder m-Dichlorbenzol, p-Xylol, Phthalid oder jeweils deren Derivate oder jeweils deren Salze bevorzugt. Als Treibstoffe sind Hydrazin oder Diesel bevorzugt.

Nachfolgend wird die Erfindung anhand nicht limitierender Figuren näher erläutert:

### KURZBESCHREIBUNG DER FIGUREN:

Figur 1 zeigt den Querschnitt durch eine schematische Darstellung einer erfindungsgemäßen Waschvorrichtung.
Figur 2 zeigt den schematischen Aufbau einer erfindungsgemäßen Reinigungsvorrichtung.
Figur 3 zeigt eine schematische Querschnittsdarstellung eines als Prallplatte ausgebildeten erfindungsgemäßen Strömungswiderstands.
Figur 4 zeigt eine schematische Darstellung eines Gitters als erfindungsgemäßen Strömungswiderstand in Form eines Querschnitts und einer teilweisen Aufsicht.
Figur 5 zeigt eine schematische Zeichnung einer Prallplatte mit verstellbaren Öffnungsquerschnitten als erfindungsgemäßen Strömungswiderstand als Querschnitt und einer teilweisen Aufsicht.
Fig. 6 zeigt einen Strömungswiderstand 6 in Aufsicht.
Fig. 7 zeigt eine schematische Zeichnung des Prallplattequerschnitt entlang der Achse I/I' der Darstellung in Fig. 6.

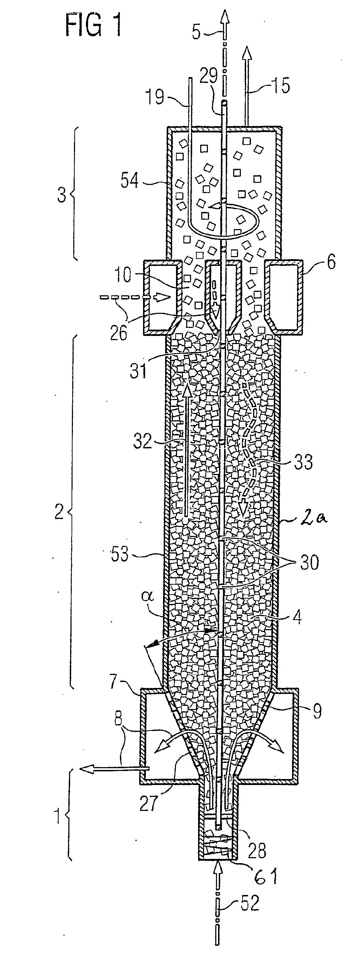

Figur 1 zeigt eine Waschvorrichtung bestehend aus einem Ersten Bereich 1, der ein Fördermittel 61, vorzugsweise ein Förderschnecke, aufweisen kann, einem Zweiten Bereich 2 und einem Dritten Bereich 3. Zwischen dem das Waschgut 4 beinhaltenden Zweiten Bereich 2 und dem Dritten Bereich 3 ist ein Strömungswiderstand 6 fest vorgesehen. Dieser Strömungswiderstand 6 ist als Prallplatte mit Öffnungen 10 ausgestaltet. Weiterhin weist der Strömungswiderstand Produktstromrückführungen 26 auf, mit denen das aufgeschmolzene Zielprodukt in den Strömungswiderstand 6 eingeleitet wird und aus den Öffnungen 10 austreten kann. Weiterhin weist der Strömungswiderstand eine Bohrung 31 zur Aufnahme eines Multithermofühlers 29 mit verschiedenen Messstellen auf. Der Zweite Bereich 2 bildet eine Waschkolonne 53 aus. Die Waschkolonne 53 ist vorzugsweise als rotationssymmetrische Röhre ausgestaltet, die genauso wie der Zweite Bereich 2 durch eine zentrale Längsachse 5 des Zweiten Bereichs 2 durchlaufen wird. Die Wandungen der Waschkolonne 53 weisen eine möglichst glatte Oberfläche auf. Die Waschkolonne 53 beinhaltet das Kristallbett 32, das die Waschkolonne 53 in Richtung des geraden Pfeils durchströmt. Entgegen der Strömungsrichtung des Kristallbetts 32 wird dieses durch eine Waschflüssigkeit 33 entsprechend der Richtung des geschlängelten Pfeils durchströmt. Zwischen dem Ersten Bereich 1 und dem Zweiten Bereich 2 ist ein Filter 7 angeordnet, der über eine Filterableitung 8 verfügt. Eine Wandung 9 des Filters 7 schließt sich an die Wandung der Waschkolonne 53 an. Die dem Waschgut 4 zugewandte Wandung des Filters 7 ist als konisches Lochblech mit Sieb ausgestaltet, wobei die Wandung des Filters 7 in einem Winkel α von >0 bis <90° zu der zentralen Längsachse des Zweiten Bereichs 5 angeordnet ist. Somit ist die Waschkolonne 53 an einem Ende durch den Strömungswiderstand 6 und an dem anderen Ende bis auf die für den Ersten Bereich bleibende Öffnung für eine Waschgutzuführung 52 durch den Filter 7 abgeschlossen. Der Erste Bereich 1 weist die als Rohr ausgebildete Waschgutzuführung 52, in der der Multithermofühler 29 durch ein Dreibein 28 fixiert ist. Der dritte Bereich 3 weist eine Aufschmelzvorrichtung 54 auf, in der eine Produktkreislaufrückführungs-Schmelze einmündet und eine Produktkreislaufrückführungs-Suspension abgeht. Die in diesem Abschnitt beschriebene Waschvorrichtung kann zum einen so betrieben werden, dass der Erste Bereich 1, wie in Fig. 1 gezeigt, unten angeordnet ist. Es ist jedoch auch möglich, die Waschvorrichtung umgekehrt zu betreiben, dadurch daß das Waschgut von oben über den Ersten Bereich 1 beaufschlagt wird.

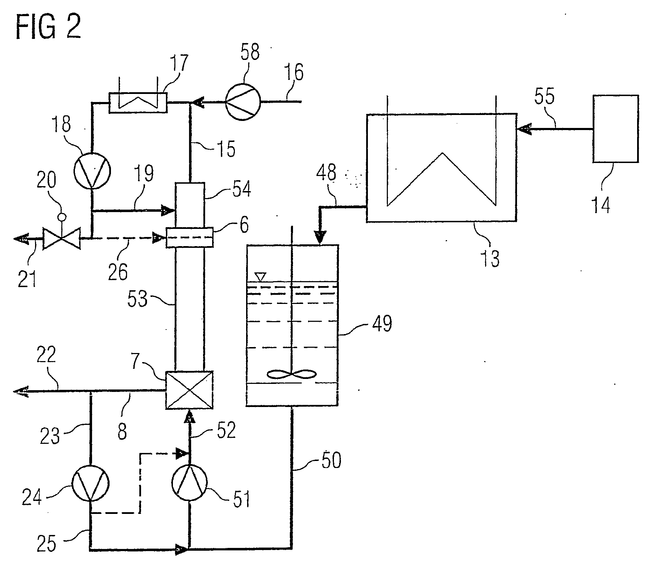

In Figur 2 wird ein in einem Reaktor, als Bestandteil einer Synthesevorrichtung 14, mit einer ggf. zwischengeschalteten Quenchvorrichtung erzeugter Feed über eine Feed-Zuleitung 55 einem Kristallerzeuger 13 zugeführt, in dem das Waschgut als Kristallsuspension erzeugt wird. Über einen Kristallüberlauf 48 wird die Kristallsuspension in einen Verweilzeitbehälter 49 überführt und über einen Verweilzeitbehälterüberlauf 50 einer Fördereinrichtung 51 für Waschgut/Suspension zugeleitet. Über eine Waschgutzuführung 52 gelangt die Kristallsuspension entlang des Filters 7 in die Waschkolonne 53 und über den Strömungswiderstand 6 in die Aufschmelzvorrichtung 54 an die sich die Produktkreislaufrückführung-Suspension 15 anschließt, die in einen Wärmetauscher 17 mündet und über eine Produktkreislaufpumpe 18 eine Rückführung über die Produktkreislaufrückführung-Schmelze 19 in die Aufschmelzvorrichtung 54 oder über die Produktstromrückführung 26 in den Strömungswiderstand 6 erfolgt. Die Menge des zurückgeführten Zielproduktes wird über ein den Produktauslass regelndes Druckventil 20 bestimmt. Das Druckventil 20 wird über das Multithermoelement 30 mittels Mehrpunktregelung angesteuert, um durch Variation der Menge des zurückgehaltenen und damit zurückgeführten Zielprodukts die Höhe der Waschfront in der Waschkolonne zu regeln. Das aufgereinigte Zielprodukt verlässt die Reinigungsvorrichtung über einen Reinproduktauslass 21. An den Reinproduktauslass 21 kann sich eine erneute Reinigungsvorrichtung anschließen, wobei das den Reinproduktauslass 21 verlassende Reinprodukt zunächst wieder in einen weiteren Kristallerzeuger, an den sich die weiteren Bestandteile der in Figur 2 dargestellten Reinigungsvorrichtung anschließen oder zumindest teilweise in den ursprünglichen Kristallerzeuger 13 zurückgeführt werden, oder beides. Ein Teil des Filtrats 22 kann über eine erste Filtratrückführung 23, betrieben über eine Filtratrückführpumpe 24 durch eine zweite Filtratrückführung 25 dem Waschgut durch Zuleitung in den Verweilzeitbehälterüberlauf 50 zugeführt werden. Bezüglich bevorzugter Verschaltungen von mehreren erfindungsgemäßen Reinigungsvorrichtungen wird auf die WO 02/055469 Bezug genommen. Zudem kann zumindest ein Teil des Filtrats 22 gemäß der mit gestrichelter Linie gekennzeichneten Leitung der Druckseite der Fördereinrichtung 51 zugeführt werden. Durch diese Maßnahme kann der Förderstrom des Waschguts bzw. der Suspension unabhängig von dem Gesamtmassenstrom eingestellt werden. Weiterhin kann vor dem Produktkreislaufwärmetauscher 17 über einen Inhibitordosierer 58 durch eine Inhibitorzuführung 16 zugeführter Inhibitor dem Zielprodukt zugeführt werden. Dieses empfiehlt sich stets dann, wenn das Zielprodukt bereits so rein ist, dass es auf Grund seiner Reaktivität zu unerwünschten und spontanen Reaktionen neigt. Weiterhin kann das Aufschmelzen der das Zielprodukt beinhaltenden Kristalle durch Bewegen einer Zielprodukt beinhaltenden Schmelze in dem durch die Bezugziffern 15, 17, 18 und 19 gebildeten Kreislauf erfolgen.

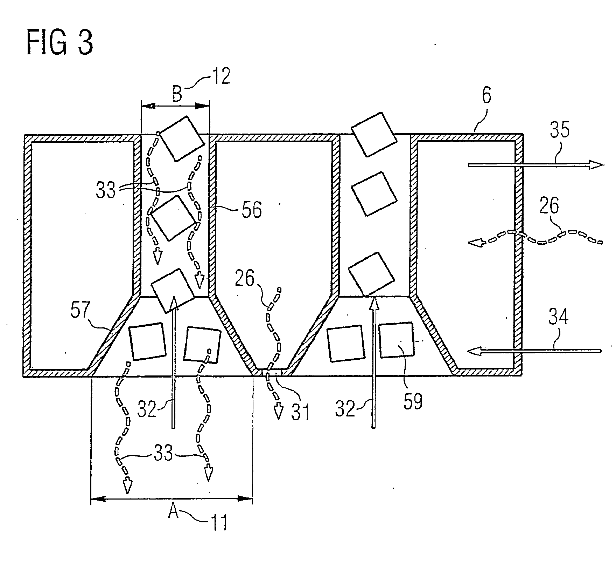

Figur 3 zeigt einen als Prallplatte ausgebildeten Strömungswiderstand 6. Diese Prallplatte weist in der Darstellungsebene zwei Öffnungen 10 auf, wobei in dem Zweiten Bereich 2 zugewandter A-Querschnitt 11 dieser Öffnungen 10 größer ist als ein dem Dritten Bereich 3 zugewandter B-Querschnitt 12. Ausgehend von dem Zweiten Bereich 2 betrachtet, besitzt die Öffnung 10 zunächst eine konische Bohrung 57 mit dem A-Querschnitt 11 an dem einem Ende und dem B-Querschnitt 12 an dem anderen Ende. An den B-Querschnitt 12 der konischen Bohrung 57 schließt sich eine Parallelbohrung 56 mit dem B-Querschnitt 12 an. Die Kristalle 59 des Kristallbetts 32 bewegen sich entlang der Richtung der geraden Pfeile durch die Öffnungen 10. Entgegengesetzt dazu fließt die Waschflüssigkeit 33 in der Richtung der geschlängelten Pfeile. Der als Prallplatte 6 ausgebildete Strömungswiderstand weist zu dessen Beheizung einen Temperiermediumeintritt 34 und ein Temperiermediumaustritt 35 auf. Weiterhin gelangen über die Produktstromrückführung 26 Produkte in die Öffnungen 10 und auf die dem Zweiten Bereich 2 zugewandten Seite des als Prallplatte ausgebildeten Strömungswiderstands 6. Durch die Bohrung 31 wird die über die Produktstromrückführung 21 in den Strömungswiderstand 6 rückgeführte Zielprodukt enthaltenden Schmelze zum Waschen der Kristalle 59 diesen zugeführt. Außerdem kann durch die Bohrung 31 das Multithermoelement 30 geführt werden.

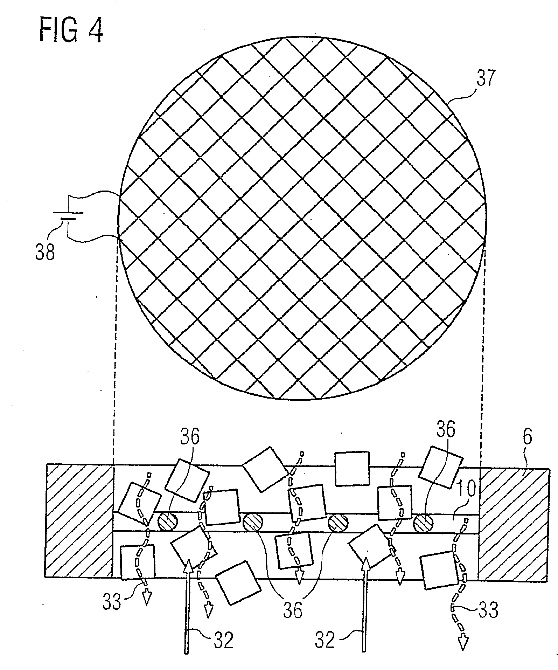

Figur 4 zeigt einen als Gitter ausgebildeten Strömungswiderstand 6, wobei die Öffnungen 10 durch die Streben 36 gebildet werden. Über eine Beheizeinrichtung 38 ist das Gitter 37 beheizbar. In einer anderen Ausgestaltung sind die Streben 36 teil eines Netzes. In den in diesem Absatz beschriebenen Ausgestaltungen ist es bevorzugt, dass die Streben 36 hohl ausgebildet sind und durch den so entstehenden Hohlraum ein Heitz/Kühl-Mittel geleitet wird, um eine möglichst überhitzungsarme Beheizung der aufzuschmelzenden Kristalle zu gewährleisten. Durch die in diesem Absatz gezeigten Ausführungen kann der Strömungswiderstand 6 beheizt werden. Bezüglich der weiteren Bezugszeichen wird auf die Ausführungen zu den vorhergehenden Figuren verwiesen.

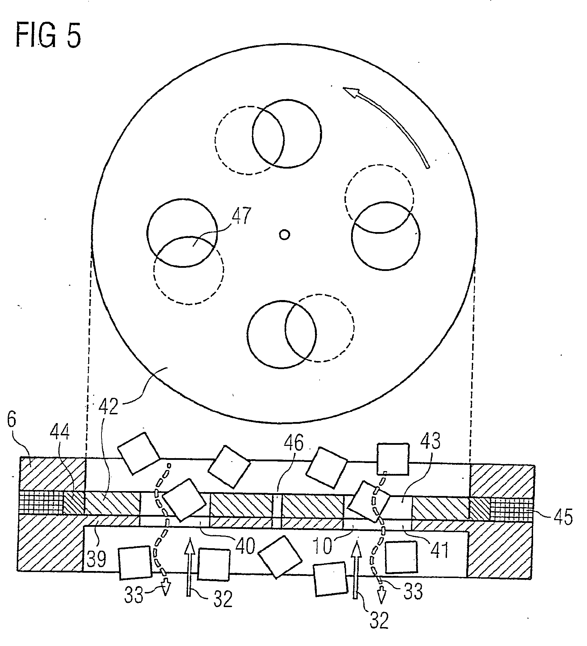

Figur 5 zeigt einen Strömungswiderstand 6 mit in ihrem Querschnitt variierbaren Öffnungen 10, aufweisend eine feste untere Lochplatte 39 und eine gegenüber der unteren Lochplatte 39 drehbewegliche Lochplatte 42, die über einen Ringmagnet 44 mit einer Antriebsspule 45 um eine Multithermofühlerführung 46 bewegbar ist. Bezüglich der weiteren Bezugszeichen wird auf die Ausführungen zu den vorhergehenden Figuren verwiesen.

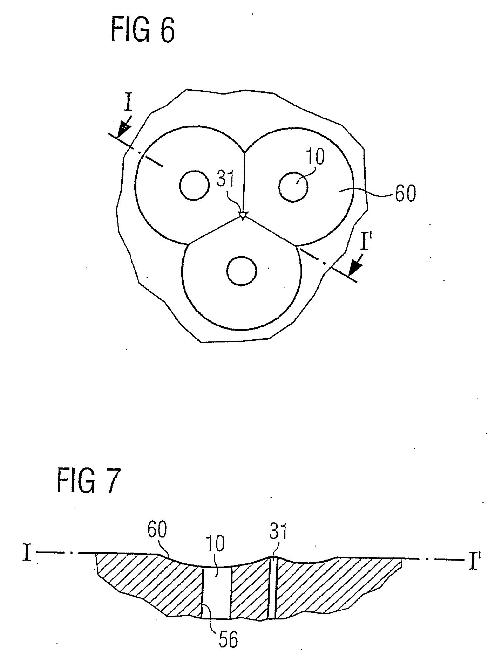

Fig. 6 zeigt einen Strömungswiderstand 6 in Aufsicht. Dieser Strömungswiderstand 6 weist mehrere Bohrungen 10 auf, die jeweils eine konvex ausgestaltete konische Bohrung 57 besitzt. Weiterhin weist dieser Ausschnitt des Strömungswiderstands 6 eine Bohrung 31 auf.

Fig. 7 zeigt einen Querschnitt entlang der Achse I/I' des in Fig. 6 abgebildeten Ausschnitts eines Strömungswiderstandes 6. Dieser Strömungswiderstand 6 weist Öffnungen 10 und 31 auf. Die Öffnung 10 wird aus einer konvexen Bohrung 60 und einer Parallelbohrung 56 gebildet, die im wesentlichen rechtwinklig zu der Oberfläche des Ausschnitts des Strömungswiderstands 6 ausgebildet sind.

Weiterhin wird die Erfindung nachfolgend durch nicht limitierende Beispiele näher erläutert.

### BEISPIELE

### Beispiel 1 (Wasseraufreinigung)

In einer Vorrichtung nach Fig. 2 mit einem 100-Liter-Kühischeibenkristaller der Firma GMF-Gouda B.V. als Kristallerzeuger 13 wurde eine 10 Gew.-%-ige Essigsäure-Wasser-Mischung in den Kristaller vorgelegt. Kristallisationswärme wurde über die Kühlflächen des Kühlscheibenkristallers abgeführt. Die Gleichgewichtstemperatur der eingesetzten Mischung betrug 3,5°C. Die bei der Kristallisation erzeugte Suspension (Suspensionsdichte ca. 30 % entsprechend einer Kristallisationstemperatur von minus 3,5°C) wurde über den Verweilzeitbehälter 49 einer Waschvorrichtung gemäß Fig. 1 (Innendurchmesser 82 mm, Länge 550 mm) beinhalten einen konischen Filter 7 (Filtermaterial 1.4571 mit 250 µm Maschenweite, unterer Durchmesser 27,7 mm, oberer Durchmesser 80,5 mm und einem Strömungswiderstand nach Fig. 3 (Höhe 60 mm mit 9 Durchgangsbohrungen á 14 mm) kontinuierlich mit einer Exzenterschneckenpumpe (150 Liter/Stunde max. Förderleistung) der Firma Netzsch GmbH zugeführt.

Zur Erzeugung der Gegenstromwäsche wurde über den reinen Produktauslaß 21 ca. 20 % des Gesamtvolumentstroms abgenommen, so dass ein Teil der Kristalle des nach oben wandernden Kristallbetts nach dem Aufschmelzen wieder durch das Kristallbett herabströmte. Auf diese Weise konnte am Kopf der Waschkolonne die Konzentration der Essigsäure auf 2,8 Gew.-% reduziert werden.

### Beispiel 2

In einer Vorrichtung nach Fig. 2 mit einem 100-Liter-Kühlscheibenkristaller (GMF-Gouda B.V.) wurde ein Acrylsäure-Gemisch der in Tabelle 1 aufgeführten Zusammensetzung vorgelegt:

**Tabelle 1**

| Name | | Feed |
|---|---|---|
| Wasser | % | 8,0 |
| Essigsäure | % | 0,052 |
| Furfural | % | 0,006 |
| Benzaldehyd | % | 0,007 |
| Acrolein | % | <0,0001 |
| Propionsäure | % | 0,028 |
| Protoanemonin | % | 0,006 |
| Acrylsäure | % | 91,625 |
| MEHQ | % | 0,016 |
| HQ | % | 0,017 |
| PZ | % | 0,036 |
| D-Acrylsäure | % | 0,227 |
| MSA | % | 0,030 |
| Sonstige | % | 0,059 |
| MEHQ | : Methyl-ethyl-hydrochinon | |
| HQ | : Hydrochinon | |
| PZ | : Phenothiazin | |
| D-Acrylsäure | : Dimere Acrylsäure | |
| MSA | : Maleinsäureanhydrid | |

Die Kristallisationswärme wurde über die Kühlflächen des Kühlscheibenkristallers abgeführt. Die Gleichgewichtstemperatur des eingesetzten Gemisches betrug 5°C. Die bei der Kristallisation erzeugte Suspension (Suspensionsdichte ca. 20 Gew.-% entsprechend einer Kristallisationstemperatur von 2°C) wurde über den Verweilzeitbehälter der Waschkolonne nach Fig. 1 (Innendurchmesser 82 mm, Länge 520 mm) beinhaltend einen konischen Filter (Filtermaterial 1.4571 mit 250 µm Maschenweite, unterer Durchmesser 27,7 mm, oberer Durchmesser 80,5 mm) und einen Strömungswiderstand nach Fig. 4 (Drahtstrebenstärke 0,5 mm, Maschenweite 1,5 mm) kontinuierlich mit einer Exzenterschneckenpumpe (300 Liter/Stunde max. Förderleistung) der Firma Netzsch GmbH zugeführt.

Zur Erzeugung der Gegenstromwäsche wurde der Reinproduktauslaß 21 vollständig abgeschlossen, so dass der gesamte Teil der Kristalle des nach oben wandernden Kristallbetts nach dem Aufschmelzen wieder durch das Kristallbett herabströmte. Auf diese Weise wurde nach 24 Stunden am Kopf der Waschkolonne eine reine Acrylsäure mit einer Zusammensetzung nach Tabelle 2 erhalten.

**Tabelle 2**

| Name | | Produkt |
|---|---|---|
| Wasser | % | 1,37 |
| Essigsäure | % | 0,013 |
| Furfural | % | <0,0001 |
| Benzaldehyd | % | 0,001 |
| Propionsäure | % | 0,01 |
| Protoanemonin | % | 0,001 |
| Acrylsäure | % | 98,558 |
| MEHQ | % | 0,001 |
| HQ | % | n.b. |
| PZ | % | n.b. |
| D-Acrylsäure | % | 0,03 |
| MSA | % | <0,001 |
| Sonstige | % | 0,009 |
| MEHQ | : Methyl-ethyl-hydrochinon | |
| HQ | : Hydrochinon | |
| PZ | : Phenothiazin | |
| D-Acrylsäure | : Dimere Acrylsäure | |
| MSA | : Maleinsäureanhydrid | |
| n.b. | : unterhalb der Messgrenze | |

Tabelle 2 zeigt, dass die erfindungsgemäße Waschvorrichtung zur Aufreinigung von Acrylsäure zu hohen Reinheiten erfolgreich eingesetzt werden kann. Das bei dieser Aufreinigung erzielte Filtrat wies eine Zusammensetzung nach Tabelle 3 auf.

**Tabelle 3**

| Name | | Filtrat |
|---|---|---|
| Wasser | % | 10,3 |
| Essigsäure | % | 0,06 |
| Furfural | % | 0,007 |
| Benzaldehyd | % | 0,008 |
| Propionsäure | % | 0,03 |
| Protoanemonin | % | 0,005 |
| Acrylsäure | % | 89,104 |
| MEHQ | % | 0,018 |
| HQ | % | 0,022 |
| PZ | % | 0,042 |
| D-Acrylsäure | % | 0,342 |
| MSA | % | 0,09 |
| Sonstige | % | 0,053 |
| MEHQ | : Methyl-ethyl-hydrochinon | |
| HQ | : Hydrochinon | |
| PZ | : Phenothiazin | |
| D-Acrylsäure | : Dimere Acrylsäure | |
| MSA | : Maleinsäureanhydrid | |

Die Konzentrationsangaben wurden mittels Gaschromatografie bestimmt. Wasserkonzentrationen wurden nach ASTM D 1364 und die Inhibitor Konzentrationen nach ASTM D 3125 bestimmt.

Das Beispiel 2 zeigt eine erhebliche Aufreinigung durch eine im erfindungsgemäßen Verfahren eingesetzte erfindungsgemäße Vorrichtung. Der Vergleich der Tabellen 1 und 2 zeigt, dass außer dem Zielprodukt Acrylsäure alle weiteren Verunreinigungen abgereichert werden konnten.

### BEZUGSZEICHENLISTE

- 1: Erster Bereich
- 2: Zweiter Bereich bzw. Säule 2a
- 3: Dritter Bereich
- 4: Waschgut
- 5: Zentrale Längsachse des Zweiten Bereichs
- 6: Strömungswiderstand
- 7: Filter
- 8: Filterableitung
- 9: Wandung
- 10: Öffnung
- 11: A-Querschnitt
- 12: B-Querschnitt
- 13: Kristallerzeuger
- 14: Syntheseeinrichtung
- 15: Produktkreislaufrückführung-Suspension
- 16: Inhibitorzuführung
- 17: Produktkreislaufwärmetauscher
- 18: Produktkreislaufpumpe
- 19: Produktkreislaufrückführung-Schmelze
- 20: Druckregelventil-Produktauslass
- 21: Reinproduktauslass
- 22: Filtrat
- 23: Erste Filtratrückführung
- 24: Filtratrückführungspumpe
- 25: Zweite Filtratrückführung
- 26: Produktstromrückführung
- 27: Konische Lochblech mit Sieb
- 28: Dreibein
- 29: Multithermofühler
- 30: Multithermoelement oder Multiwiderstandsthermometer
- 31: Bohrung
- 32: Kristallbett
- 33: Waschflüssigkeit
- 34: Temperiermediumeintritt
- 35: Temperiermediumaustritt
- 36: Streben
- 37: Gitter mit unterschiedlicher Maschenweite
- 38: Beheizeinrichtung
- 39: Untere Lochplatte
- 40: Öffnungen untere Lochplatte
- 41: Anfasung der Öffnung
- 42: Obere Lochplatte
- 43: Öffnungen obere Lochplatte
- 44: Ringmagnet
- 45: Antriebsspule
- 46: Multithermofühlerführung
- 47: Freie Querschnittsflächen
- 48: Kristallerüberlauf
- 49: Verweilzeitbehälter
- 50: Verweilzeitbehälterüberlauf
- 51: Fördereinrichtung für Waschgut/Suspension
- 52: Waschgutzuführung
- 53: Waschkolonne
- 54: Aufschmelzvorrichtung
- 55: Feed-Leitung
- 56: Parallelbohrung
- 57: Konische Bohrung
- 58: Inhibitordosierer
- 59: Kristalle
- 60: konvexe Bohrung
- 61: Fördermittel bzw. Förderschnecke

## Patentansprüche

1. Waschvorrichtung, aufweisend
- einen Ersten Bereich (1), für die Zuführung eines Waschguts (4),
- einen Zweiten Bereich (2), für die Waschung des Waschguts (4), und
- einen Dritten Bereich (3), zur Aufschmelzung des Waschguts (4), sowie
- einen Strömungswiderstand (6), der zwischen dem Zweiten Bereich (2) und dem Dritten Bereich (3) vorgesehen ist,
**dadurch gekennzeichnet, dass**
der Zweite Bereich (2) mindestens teilweise aus einer Säule (2a) gebildet wird, wobei diese Säule einen Durchmesser von mindestens 300 mm aufweist
und zwischen dem Ersten Bereich (1) und dem Zweiten Bereich (2) eine Fest/Flüssig-Trennvorrichtung vorgesehen ist.

2. Waschvorrichtung nach Anspruch 1, wobei der Strömungswiderstand um eine zentrale Längsachse (5) des Zweiten Bereichs (2) drehfest vorgesehen ist.

3. Waschvorrichtung nach Anspruch 1 oder 2, **dadurch gekennzeichnet, dass** als Fest/Flüssig-Trennvorrichtung, ein Filter (7), mit einer Filtratableitung (8) vorgesehen ist.

4. Waschvorrichtung nach Anspruch 3, wobei die Fest/Flüssig-Trennvorrichtung als ein Filter (7) in einer an dem Zweiten Bereich (2) anschließenden Wandung (9) ausgebildet ist.

5. Waschvorrichtung nach Anspruch 4, wobei der die Wandung (9) mit einem Winkel α im Bereich von >0 und <90°, bezogen auf die zentrale Längsachse (5) angeordnet ist.

6. Waschvorrichtung nach einem der vorhergehenden Ansprüche, wobei der Strömungswiderstand (6) mindestens eine Öffnung (10) aufweist.

7. Waschvorrichtung nach einem der vorhergehenden Ansprüche, wobei der Strömungswiderstand (6) durch eine relative freie Querschnittsfläche im Bereich von >0 bis <100 %, bezogen auf die Gesamtfläche des Strömungswiederstands, gekennzeichnet ist.

8. Waschvorrichtung nach Anspruch 7, wobei die freie Querschnittsfläche variierbar ist.

9. Waschvorrichtung nach einem der Ansprüche 7 oder 8, wobei Strömungswiderstand (6) temperierbar ist.

10. Waschvorrichtung nach einem der vorhergehenden Ansprüche, wobei vor dem Ersten Bereich (1) oder mindestens teilweise in dem Ersten Bereich (1) ein pulsationsarmes Fördermittel (61) vorgesehen ist.

11. Waschvorrichtung nach Anspruch 10, wobei das pulsationsarme Fördermittel (61) eine Förderschnecke (61) aufweist.

12. Reinigungsvorrichtung, aufweisend einen mit der in einem der vorstehenden Ansprüche definierten Waschvorrichtung kristallleitend verbundenen Kristallerzeuger (13).

13. Reinigungsvorrichtung nach Anspruch 12, wobei zwischen dem Kristallerzeuger (13) und der Waschvorrichtung ein Verweilzeitbehälter (49) vorgesehen ist.

14. Synthesevorrichtung, aufweisend eine Syntheseeinrichtung (14), und stromab eine nach einem der Ansprüche 12 oder 13 definierte Reinigungsvorrichtung.

15. Synthesevorrichtung nach Anspruch 14, wobei die Syntheseeinrichtung (14) eine Gasphasenoxidationssyntheseeinheit ist.

16. Verfahren zur Aufreinigung von einem Waschgut, wobei das Waschgut über den Ersten Bereich (1) einer in einem der Ansprüche 1 bis 11 definierten Waschvorrichtung zugeführt und ein Zielprodukt erhalten wird.

17. Verfahren nach Anspruch 16, wobei das Waschgut mindestens 20 Gew.-% des Zielproduktes beinhaltet.

18. Verwendung des nach einem Verfahren nach Anspruch 16 oder 17 erhaltene Zielprodukt bei der Herstellung von Lebensmitteln, Polymeren, Treibstoffen, Schmiermitteln, Reinigungsmitteln, Farbstoffen oder Arzneimitteln.

19. Verwendung einer Waschvornchtung nach einem der Ansprüche 1 bis 11 oder einer Reinigungsvorrichtung nach Anspruch 12 zur Aufreinigung von Zielprodukten wie Lebensmittel, Monomere, Treibstoffe, Lösemittel; oder zur Abwasseraufarbeitung; oder zur Isomerentrennung.

## Claims

1. Washing device, comprising:
- a First Region (1), for supplying a washing product (4),
- a Second Region (2), for washing the washing product (4), and
- a Third Region (3), for melting the washing product (4), and
- a flow resistance (6), which is provided between the Second Region (2) and the Third Region (3),
**characterized in that** the Second Region (2) is at least partially in the form of a column (2a), wherein this column has a diameter of at least 300 mm,
and a solid/liquid separating device is provided between the First Region (1) and the Second Region (2).

2. Washing device according to Claim 1, the flow resistance being provided for conjoint rotation about a central longitudinal axis (5) of the Second Region (2).

3. Washing device according to Claim 1 or 2, **characterized in that** a filter (7) with a filtrate offtake line (8) is provided as the solid/liquid separating device.

4. Washing device according to Claim 3, wherein the solid/liquid separating device is in the form of a filter (7) in a wall (9) adjoining the Second Region (2).

5. Washing device according to Claim 4, wherein the wall (9) is arranged at an angle α in the range of > 0 and < 90°, relative to the central longitudinal axis (5).

6. Washing device according to one of the preceding claims, wherein the flow resistance (6) has at least one opening (10).

7. Washing device according to one of the preceding claims, wherein the flow resistance (6) is **characterized by** a relative free cross-sectional area in the range of > 0 to < 100%, relative to the total area of the flow resistance.

8. Washing device according to Claim 7, wherein the free cross-sectional area is variable.

9. Washing device according to either of Claims 7 and 8, wherein the temperature of the flow resistance (6) can be controlled.

10. Washing device according to one of the preceding claims, wherein a low-pulsation conveying means (61) is provided upstream of the First Region (1) or at least partially in the First Region (1).

11. Washing device according to Claim 10, wherein the low-pulsation conveying means (61) comprises a conveyor screw (61).

12. Purification device, comprising a crystal producer (13) connected in a crystal-carrying manner to the washing device defined in one of the preceding claims.

13. Purification device according to Claim 12, wherein a dwell-time container (49) is provided between the crystal producer (13) and the washing device.

14. Synthesis device, comprising a synthesis unit (14), and downstream a purification device defined according to either of Claims 12 and 13.

15. Synthesis device according to Claim 14, wherein the synthesis unit (14) is a gaseous phase oxidation synthesis unit.

16. Method for purifying a washing product, wherein the washing product is supplied by way of the First Region (1) to a washing device defined in one of Claims 1 to 11 and a target product is obtained.

17. Method according to Claim 16, wherein the washing product contains at least 20% by weight of the target product.

18. Use of the target product obtained by a method according to Claim 16 or 17 in the production of foods, polymers, fuels, lubricants, cleaning agents, dyestuffs or pharmaceuticals.

19. Use of a washing device according to one of Claims 1 to 11 or a purification device according to Claim 12 for purifying target products such as foods, monomers, fuels, solvents; or for waste-water treatment; or for isomer separation.

## Revendications

1. Dispositif de lavage, présentant :
- une première région (1) pour l'introduction d'un produit à laver (4),
- une deuxième région (2) pour le lavage du produit à laver (4), et
- une troisième région (3) pour la fusion du produit à laver (4), ainsi
- qu'une résistance à l'écoulement (6) qui est prévue entre la deuxième région (2) et la troisième région (3),
**caractérisé en ce que** la deuxième région (2) est formée au moins en partie d'une colonne (2a), cette colonne présentant un diamètre d'au moins 300 mm et
entre la première région (1) et la deuxième région (2) étant prévu un dispositif de séparation des matières solides/fluides.

2. Dispositif de lavage selon la revendication 1, dans lequel la résistance à l'écoulement est prévue de manière solidaire en rotation autour d'un axe longitudinal central (5) de la deuxième région (2).

3. Dispositif de lavage selon la revendication 1 ou 2, **caractérisé en ce que** l'on prévoit en tant que dispositif de séparation des matières solides/fluides un filtre (7) avec une conduite (8) d'évacuation du filtrat.

4. Dispositif de lavage selon la revendication 3, dans lequel le dispositif de séparation des matières solides/fluides est réalisé sous la forme d'un filtre (7) dans une paroi (9) se raccordant à la deuxième région (2).

5. Dispositif de lavage selon la revendication 4, dans lequel la paroi (9) est disposée avec un angle α dans une plage de > 0 et < 90° par rapport à l'axe longitudinal central (5).

6. Dispositif de lavage selon l'une quelconque des revendications précédentes, dans lequel la résistance à l'écoulement (6) présente au moins une ouverture (10).

7. Dispositif de lavage selon l'une quelconque des revendications précédentes, dans lequel la résistance à l'écoulement (6) est **caractérisée par** une surface en section transversale relativement libre dans une plage de > 0 à < 100 %, par rapport à la surface totale de la résistance à l'écoulement.

8. Dispositif de lavage selon la revendication 7, dans lequel la surface en section transversale libre est variable.

9. Dispositif de lavage selon l'une quelconque des revendications 7 ou 8, dans lequel la résistance à l'écoulement (6) peut être régulée en température.

10. Dispositif de lavage selon l'une quelconque des revendications précédentes, dans lequel un moyen de transport à faible pulsation (61) est prévu avant la première région (1) ou au moins en partie dans la première région (1).

11. Dispositif de lavage selon la revendication 10, dans lequel le moyen de transport à faible pulsation (61) présente une vis transporteuse (61).

12. Dispositif de nettoyage présentant un générateur de cristaux (13) connecté de manière conduisant les cristaux au dispositif de lavage selon l'une quelconque des revendications précédentes.

13. Dispositif de nettoyage selon la revendication 12, dans lequel un récipient de temporisation (49) est prévu entre le générateur de cristaux (13) et le dispositif de lavage.

14. Dispositif de synthèse présentant un système de synthèse (14) et, en aval de celui-ci, un dispositif de nettoyage selon l'une quelconque des revendications 12 ou 13.

15. Dispositif de synthèse selon la revendication 14, dans lequel le système de synthèse (14) est une unité de synthèse à oxydation en phase gazeuse.

16. Procédé de nettoyage d'un produit à laver, le produit à laver étant acheminé par le biais de la première région (1) à un dispositif de lavage selon les revendications 1 à 11 et un produit cible étant obtenu.

17. Procédé selon la revendication 16, dans lequel le produit à laver contient au moins 20 % en poids du produit cible.

18. Utilisation du produit cible obtenu selon un procédé selon la revendication 16 ou 17 pour la fabrication d'aliments, de polymères, de charges propulsives, de lubrifiants, de détergents, de peintures ou de médicaments.

19. Utilisation d'un dispositif de lavage selon l'une quelconque des revendications 1 à 11, ou d'un dispositif de nettoyage selon la revendication 12 pour le nettoyage de produits cibles tels que des aliments, des monomères, des charges propulsives, des solvants ; ou pour le traitement d'eaux usées ; ou pour la séparation d'isomères.
